# EUROPEAN PATENT SPECIFICATION

(11) **EP 0 367 596 B1**
(45) Date of publication and mention of the grant of the patent: **22.12.1993**
(21) Application number: 89311322.5
(22) Date of filing: 01.11.1989
(51) Int. Cl.: A61K 39/395

(54) **Antagonist to interleukin-5 for preventing or reducing eosinophilia**
Antagonist für Interleukin-5 zur Verhütung oder Verminderung von Eosinophilie
Antagoniste de l'interleukin-5 pour prévenir ou réduire l'éosinophilie

(30) Priority: 03.11.1988 US 266909
(43) Date of publication of application: 09.05.1990
(73) Proprietor: SCHERING CORPORATION, Kenilworth New Jersey 07033 (US)
(72) Inventor: Coffman, Robert L., Portola Valley, CA 94025 (US); Rennick, Donna M., Los Altos, CA 94022 (US)
(74) Representative: Ritter, Stephen David

(56) References cited:
- JOURNAL OF EXPERIMENTAL MEDICINE, vol. 167, January 1988, pp. 219-224#
- JOURNAL OF IMMUNOLOGY, vol. 141, no. 5, September 1988; pp. 1576-1581#

## Description

### Field of the Invention

The invention relates generally to treating diseases associated with elevated populations of eosinophils, and more particularly to inhibiting eosinophil production and accumulation by blocking the stimulatory effects of interleukin-5 (IL-5).

### BACKGROUND

Eosinophils are white blood cells of the granulocytic lineage. Their normal function appears to be combating parasitic infections, particularly helminthic infections. However, their accumulation in tissues, a condition referred to as eosinophilia, is also associated with several disease states, most notably asthma; e.g. Frigas et al., J. Allergy and Clinical Immunol., Vol. 77, pgs. 527-537 (1986); Gleich, Hospital Practice (March 15, 1988); and Weller, J. Allergy and Clinical Immunol., Vol. 73, pgs. 1-10 (1984). It is believed that the damage to the epithelial lining of the bronchial passages in severe asthmatic attacks is largely caused by the compounds released by degranulating eosinophils.

Currently glucocorticoid steroids are the most effective drugs for treating the acute effects of allergic diseases, such as asthma. However, prolonged steroid treatment is associated with many deleterious side effects; Goodman and Gillman, The Pharmacological Basis of Therapeutics, 6th Ed. (MacMillan Publishing Company, New York, 1980). Moreover, the steroids apparently do not affect the production or accumulation of granulocytic cells, such as eosinophils, in the afflicted tissues. The availability of alternative or complementary approaches to the treatment of disorders associated with eosinophilia would have important clinical utility.

In J. Exp. Med., Vol. 167, 1988 (January 1988), p. 219-224, in a report by Lopez et al, it is disclosed that IL-5 is one of four cytokines that are known to stimulate eosinophil proliferation and/or function. IL-5 was shown to induce morphological changes on and functional activation of eosinophils.

### SUMMARY OF THE INVENTION

The invention is the use of an antagonist to human Interleukin-5 (IL-5) in the manufacture of a pharmaceutical composition for preventing or reducing eosinophilia in a patient.

Preferably, the antagonists to IL-5 are monoclonal antibodies, or binding compositions derived therefrom by standard techniques.

### BRIEF DESCRIPTION OF THE DRAWINGS

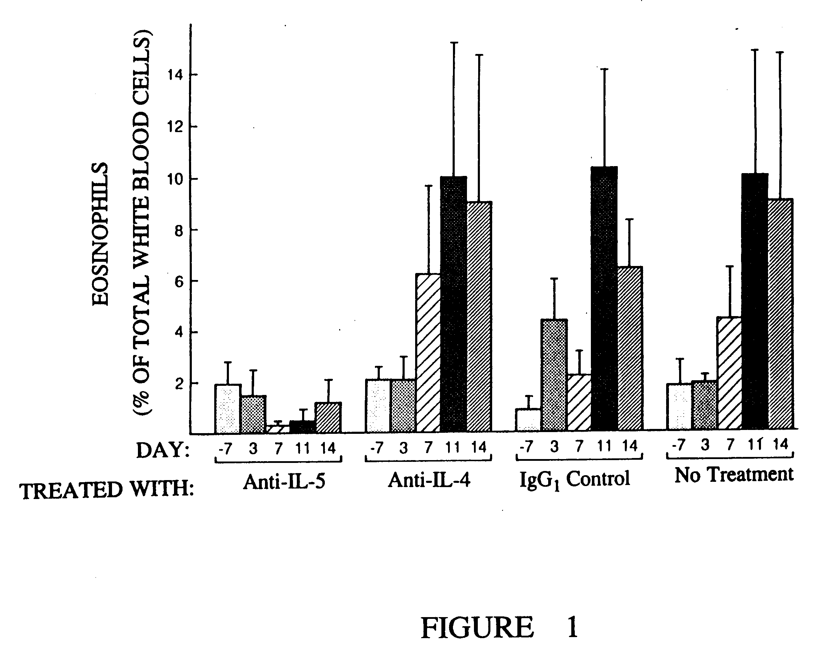

Figure 1 graphically illustrates data on peripheral blood levels of eosinophils (as percentage of total white blood cells) in parasite-infected mice with and without treatment with a blocking IL-5 monoclonal antibody.

### DETAILED DESCRIPTION OF THE INVENTION

The invention is based on the discovery that IL-5 increases the production of eosinophils and that antagonists of Il-5 reduce the production of eosinophils and their accumulation in tissues. The use of the invention according to the claims comprises administering to a patient an effective, or disease-ameliorating amount, of an antagonist to human IL-5.

Preferably, antagonists of the invention are derived from antibodies specific for human IL-5. More preferably, the antagonists of the invention comprise fragments or binding compositions specific for IL-5.

Antibodies comprise an assembly of polypeptide chains linked together by disulfide bridges. Two major polypeptide chains, referred to as the light chain and the heavy chain, make up all major structural classes (isotypes) of antibody. Both heavy chains and light chains are further divided into subregions referred to as variable regions and constant regions. Heavy chains comprise a single variable region and three different constant regions, and light chains comprise a single variable region (different from that of the heavy chain) and a single constant region (different from those of the heavy chain). The variable regions of the heavy chain and light chain are responsible for the antibody's binding specificity.

As used herein, the term "heavy chain variable region" means a polypeptide (1) which is from 110 to 125 amino acids in length, and (2) whose amino acid sequence corresponds to that of a heavy chain of a monoclonal antibody of the invention, starting from the heavy chain's N-terminal amino acid. Likewise, the term "light chain variable region" means a polypeptide (1) which is from 95 to 115 amino acids in length, and (2) whose amino acid sequence corresponds to that of a light chain of a monoclonal antibody of the invention, starting from the light chain's N-terminal amino acid.

As used herein the term "monoclonal antibody" refers to homogenous populations of immunoglobulins which are capable of specifically binding to human IL-5.

As used herein the term "binding composition" means a composition comprising two polypeptide chains (1) which, when operationally associated, assume a conformation having high binding affinity for human interleukin-5, and (2) which are derived from a hybridoma producing monoclonal antibodies specific for human interleukin-5. The term "operationally associated" is meant to indicate that the two polypeptide chains can be positioned relative to one another for binding by a variety of means, including association in a native antibody fragment, such as Fab or Fv, or by way of genetically engineered cysteine-containing peptide linkers at the carboxyl termini. Normally, the two polypeptide chains correspond to the light-chain variable region and heavy-chain variable region of a monoclonal antibody specific for human interleukin-5. Preferably, the binding compositions are derived from monoclonal antibodies of the IgA isotype, so that the naturally occurring cysteine linkage between the light- and heavy-chain variable regions can be used to bring the chains into operable association.

Preferably, antagonists of the invention are derived from monoclonal antibodies specific for human IL-5. Monoclonal antibodies capable of blocking IL-5 are selected by their ability to inhibit IL-5-induced effects in standard IL-5 bioassays, such as the ability to stimulate the growth and development of eosinophils in in vitro colony-forming assays, and the ability to augment in vitro proliferation of the in vivo passaged BCL₁ lymphoma cells. The former assay is described in several references, e.g. Todd-Sanford, Clinical Diagnosis by Laboratory Methods, 15th Ed., Davidson and Henry, eds. 1974). The latter assay is described by Hamiblin and O'Garra, pgs. 209-228, in Lymphocytes: A Practical Approach, Klaus ed. (IRL Press, Oxford, 1987). BCL₁ cells are available from the ATCC under accession number TIB 197 and are described in Nature, Vol. 272, pgs. 624-626 (1978), in Immunol. Rev., Vol. 48, pgs. 169-195 (1979), and in J. Immunol., Vol. 125, pgs. 976-980 (1980).

Briefly, the eosinophil assay can be performed as follows using either bone-marrow cells or umbilical-cord blood cells. Bone-marrow cells collected from patients with nonhematologic disease are layered over Ficoll® (type 400, Sigma Chemical Co., St. Louis, MO) and centrifuged (600 x g), and the cells at the interface are removed. These cells are washed twice in Iscove's Modified Dulbecco's Medium containing 10% fetal calf serum (FCS) and resuspended in the same medium, and the adherent cells are removed by adherence to plastic Petri dishes. The nonadherent cells are added at 10⁵ cells/ml to Iscove's Medium containing 20% FCS, 50 µM 2-mercaptoethanol, 0.9% methylcellulose and varied concentrations of either supernatants known to contain colony stimulating activity or test supernatants. One ml aliquots are plated in 35 mm petri dishes and cultured at 37°C in a fully humidified atmosphere of 6% CO₂ in air. Three days after the initiation of the culture, 1 unit of erythropoietin is added to each plate. Granulocyte-macrophage colonies and erythroid bursts are scored at 10-14 days using an inverted microscope.

Cord-blood cells collected in heparin are spun at 600 x g. The white blood cells at the interface between the plasma and red blood cell peak are transferred to a tube containing 0.17 N ammonium chloride and 6% FCS. After 5 min on ice, the suspension is underlaid with 4 ml FCS and centrifuged at 600 x g. The cell pellet is washed with Dulbecco's phosphate buffered saline and put through the Ficoll® and plastic adherence steps as described above for bone-marrow cells. The low-density nonadherent cells are collected and placed at 10⁵ cells/culture in the semi-solid culture medium as described above.

At the end of the assays, the cellular composition is determined after applying the individual colonies to glass slides and staining with Wright-Geimsa. As mentioned above, eosinophils are determined by staining with Luxol Fast Blue (Johnson, G. and Metcalf, D., Exp. Hematol. Vol. 8, pgs. 549-561 [1980]).

Briefly, for the BCL₁-based assay, spleens are removed from the mice (preferably BALB/cByJ or BALB/cdJ) bearing the BCL₁ tumor (recoveries vary from 8 x 10⁸ to 1.3 x 10⁹ cells per mouse), and a cell suspension is prepared and treated with anti-Thy-1 monoclonal antibodies and complement to deplete T cells (e.g. one spleen in 10 ml of RPMI + 5% fetal calf serum, 5 ml of 1:3 guinea pig complement, and 0.5 ml of a pre-treated anti-Thy-1). Cells in the same medium (2.5 x 10⁵ cells/ml) are plated out into microtiter cultures in 100 µl volumes. The compounds to be tested for IL-5 activity are added to the cultures in 100 µl of medium. The cultures are incubated are incubated at 37°C for 2 days and then assayed for DNA synthesis.

Hybridomas of the invention are produced by well-known techniques. Usually, an immortalizing cell line is fused with a B-lymphocyte that produces the desired antibody. Alternatively, non-fusion techniques for generating immortal antibody-producing cell lines are possible, and come within the purview of the present invention, e.g. virally induced transformation: Casali et al., "Human Monoclonals from Antigen-Specific Selection of B Lymphocytes and Transformation by EBV," Science, Vol. 234, pgs. 476-479 (1986). Immortalizing cell lines are usually transformed mammalian cells, particularly myeloma cells of rodent, bovine, and human origin. Most frequently, rat or mouse myeloma cell lines are employed as a matter of convenience and availability.

Techniques for obtaining the appropriate lymphocytes from mammals injected with the target antigen are well known. Generally, either peripheral blood lymphocytes (PBLs) are used if cells of human origin are desired, or spleen cells or lymph node cells are used if non-human mammalian sources are desired. A host mammal is injected with repeated dosages of the purified antigen, and the mammal is permitted to generate the desired antibody-producing cells before these are harvested for fusion with the immortalizing cell line. Techniques for fusion are also well known in the art, e.g. mixing the cells with a fusing agent, such as polyethylene glycol. Hybridomas are selected by standard procedures, such as HAT selection. From among these hybridomas, those secreting the desired antibody are selected by assaying their culture medium by standard immunoassays, such as Western blotting, ELISA, RIA, or the like. Antibodies are recovered from the medium using standard protein purification techniques: e.g. Tijssen, Practice and Theory of Enzyme Immunoassays (Elsevier, Amsterdam, 1985). Many references are available for guidance in applying any of the above techniques, e.g. Kohler et al., Hybridoma Techniques (Cold Spring Harbor Laboratory, New York, 1980); Tijssen, Practice and Theory of Enzyme Immunoassays (Elsevier, Amsterdam, 1985); Campbell, Monoclonal Antibody Technology (Elsevier, Amsterdam, 1984); Hurrell, Monoclonal Hybridoma Antibodies: Techniques and Applications (CRC Press, Boca Raton, FL, 1982); and the like.

The use and generation of fragments of antibodies is also well known, e.g. Fab fragments: Tijssen, Practice and Theory of Enzyme Immunoassays (Elsevier, Amsterdam, 1985); and Fv fragments: Hochman et al., Biochemistry, Vol. 12, pgs. 1130-1135 (1973), Sharon et al., Biochemistry, Vol. 15, pgs. 1591-1594 (1976) and Erhlich et al., U.S. Patent 4,355,023; and antibody half molecules: Auditore-Hargreaves, U.S. Patent 4,470,925. Moreover, such compounds and compositions of the invention can be used to construct bi-specific antibodies by known techniques, e.g., by further fusions of hybridomas (i.e. to form so-called quadromas): Reading, U.S. Patent 4,474,493; or by chemical reassociation of half molecules: Brennan et al., Science, Vol. 229, pgs. 81-83 (1985).

Hybridomas and monoclonal antibodies of the invention are produced against either glycosylated or unglycosylated versions of recombinantly-produced mature human interleukin-5. Generally, unglycosylated versions of human IL-5 are produced in E. coli, and glycosylated versions are produced in mammalian cell hosts, e.g. CV1 or COS monkey cells, mouse L cells, or the like. Recombinantly-produced mature human IL-5 is produced by introducing an expression vector into a host cell using standard protocols: e.g. Maniatis et al., Molecular Cloning: A Laboratory Manual (Cold Spring Harbor Laboratory, New York, 1982); Okayama and Berg, Mol. Cell. Biol., Vol. 2, pgs. 161-170 (1982) and Vol. 3, pgs. 280-289 (1983); Hamer, Genetic Engineering, Vol. 2, pgs. 83-100 (1980) and U.S. Patent 4,599,308; Kaufman et al., Mol. Cell. Biol., Vol. 2, pgs. 1304-1319 (1982); or the like.

Construction of bacterial or mammalian expression vectors is well known in the art, once the nucleotide sequence encoding a desired protein is known or otherwise available; e.g. DeBoer in U.S. Patent 4,551,433 discloses promoters for use in bacterial expression vectors; Goeddel et al. in U.S. Patent 4,601,980, and Riggs in U.S. Patent 4,431,739 disclose the production of mammalian proteins by E. coli expression systems; and Riggs (cited above), Ferretti et al., Proc. Natl. Acad. Sci., Vol. 83, pgs. 599-603 (1986), Sproat et al., Nucleic Acids Research, Vol. 13, pgs. 2959-2977 (1985), and Mullenbach et al., J. Biol. Chem., Vol. 261, pgs. 719-722 (1986) disclose how to construct synthetic genes for expression in bacteria. Accordingly, these references are incorporated by reference. The amino acid sequence of mature human IL-5 is disclosed by Azuma et al., Nucleic Acids Research, Vol. 14, pgs. 9145-9158 (1986), and synthetic genes encoding human IL-5 are available commercially from Beckman Instruments (Fullerton, CA). Many bacterial expression vectors and hosts are available commercially and through the ATCC.

Antibodies and antibody fragments characteristic of hybridomas of the invention can also be produced by recombinant means by extracting messenger RNA, constructing a cDNA library, and selecting clones which encode segments of the antibody molecule: e.g. Wall et al., Nucleic Acids Research, Vol. 5, pgs. 3113-3128 (1978); Zakut et al., Nucleic Acids Research, Vol. 8, pgs. 3591-3601 (1980); Cabilly et al., Proc. Natl. Acad. Sci., Vol. 81, pgs. 3273-3277 (1984); Boss et al,, Nucleic Acids Research, Vol. 12, pgs. 3791-3806 1984); Amster et al., Nucleic Acids Research, Vol. 8, pgs. 2055-2065 (1980); Moore et al., U.S. Patent 4,642,334; and Skerra et al., Science, Vol. 240, pgs. 1038-1041 (1988). In particular, such techniques can be used to produce interspecific monoclonal antibodies, wherein the binding region of one species is combined with the non-binding region of the antibody of another species to reduce immunogenicity; e.g. Liu et al., Proc. Natl. Acad. Sci., Vol. 84, pgs. 3439-3442 (1987).

Antagonists of the invention are administered as a pharmaceutical composition. Such compositions contain a therapeutic amount of at least one of the monoclonal antibodies of the invention, or fragments thereof, in a pharmaceutically effective carrier. A pharmaceutical carrier can be any compatible, non-toxic substance suitable for delivering the compositions of the invention to a patient. Sterile water, alcohol, fats, waxes, and inert solids may be included in a carrier. Pharmaceutically acceptable adjuvants (e.g. buffering agents, dispersing agents) may also be incorporated into the pharmaceutical composition. Generally, compositions useful for parenteral administration of such drugs are well known, e.g. Remington's Pharmaceutical Science, 15th Ed. (Mack Publishing Company, Easton, PA 1980). Alternatively, compositions of the invention may be introduced into a patient's body by an implantable drug delivery system, e.g. Urquhart et al., Ann. Rev. Pharmocol. Toxicol., Vol. 24, pgs. 199-236 (1984).

When the antagonists of the invention are derived from antibodies, they are normally administered parenterally, preferably intravenously. Since such protein or peptide antagonists may be immunogenic, they are preferably administered slowly, either by a conventional IV administration set or from a subcutaneous depot, e.g. as taught by Tomasi et al., U.S. patent 4,732,863.

When administered parenterally the antibodies or fragment will be formulated in a unit dosage injectable form (typically in solution, suspension or emulsion) in association with a pharmaceutically acceptable parenteral vehicle. Such vehicles are inherently nontoxic and nontherapeutic. Examples of such vehicles are normal saline, Ringer's solution, dextrose solution, and Hank's solution. Nonaqueous vehicles such as fixed oils and ethyl oleate may also be used. A preferred vehicle is 5% dextrose/saline. The vehicle may contain minor amounts of additives such as substances that enhance isotonicity and chemical stability, e.g. buffers and preservatives. The antibody is preferably formulated in purified form substantially free of aggregates and other proteins at concentrations of about 5 to 30 mg/ml, preferably 10 to 20 mg/ml.

Selecting an administration regimen for an antagonist depends on several factors, including the serum turnover rate of the antagonist, the serum level of IL-5 associated with the eosinophilia, the immunogenicity of the antagonist, the accessibility of the target IL-5 (e.g. if non-serum Il-5 is to be blocked), the affinity of IL-5 to its receptor(s) relative to that of IL-5 to the antagonist, and the like. Preferably, an administration regimen maximizes the amount of antagonist delivered to the patient consistent with an acceptable level of side effects. Accordingly, the amount of antagonist delivered depends in part on the particular antagonist and the severity of the disease being treated. Guidance in selecting appropriate doses is found in the literature on therapeutic uses of antibodies: e.g. Bach et al., chapter 22, in Ferrone et al., eds., Handbook of Monoclonal Antibodies (Noges Publications, Park Ridge, NJ, 1985); and Russell, pp 303-357, and Smith et al., pgs. 365-389, in Haber et al., eds., Antibodies in Human Diagnosis and Therapy (Raven Press, New York, 1977). Preferably, whenever the antagonist comprises monoclonal antibodies or Fab-sized fragments thereof (including binding compositions), the dose is in the range of about 1-20 mg/kg per day, more preferably about 1-10 mg/kg per day.

### EXAMPLES

The following Examples serve to illustrate aspects of the present invention. The selected vectors, hosts, fusion partners as well as concentration of reagents, temperatures, and the values of other variables are to exemplify the invention.

### Example I. Production of IL-5-Blocking Monoclonal Antibody TRFK-5

Production of the TRFK-5 hybridoma is described by Schumacher et al., J. Immunol., Vol. 141, pgs. 1576-1581 (1988). Two 12-week old Lewis rats received three injections each of 0.4 to 1.6 µg of purified mouse IL-5 in Complete Freund's Adjuvant 4 to 5 weeks apart. Both rats were bled at 10 and 18 days after the third injection. The rat demonstrating the higher blocking serum titer received a final i.v. injection of 2 µg of purified mouse IL-5. Three days later, its spleen and popliteal lymph node cells were fused using P3X63Ag8.653 myeloma cells (available from the American Type Culture Collection under accession number CRL 1580) by the method of Kipps and Herzenberg in Vol. 4 of the Handbook of Experimental Immunology, Weir, ed. (Blackwell Scientific Publications, Oxford, 1986). Red blood cells were lysed in distilled water, and their physiologic osmolarity was immediately restored with HBSS (Hank's balanced salt solution). After fusion using 50% polyethylene glycol (M.W. 1500 to 1800, available from Sigma Chemical Co.), cells were plated at a density of 8.5 x 10⁵ cells/ml, 100 µl/well, in HAT selection medium. The next day, 150 µl of HAT medium were added to each well, and the medium was refreshed at 5 and 8 days. At 10 days the medium was changed to 100 µM hypoxanthine/16 µM thymidine medium, and the cells were maintained in this medium for an additional 14 days. Fourteen days after fusion, hybridoma supernatants were screened by direct blocking of the growth-stimulating activity of 0.5 ng/ml of mouse IL-5 in the BCL₁ assay. Of 286 wells containing hybridoma growth (43% total wells), four hybridomas producing consistent antibody activity were cloned by limiting dilution in maintenance medium until stable subclones were isolated. One of these, designated TRFK-5, produced antibodies that were able to block both mouse and human IL-5, and was chosen for deposit.

IL-5 used as the immunogen was purified from supernatant of the T cell clone MB2-1, described by Giedlin et al., Cell. Immunol., Vol. 97, pg. 357 (1986), by a procedure described by Bond et al., J.Immunol., Vol. 139, pg. 3691 (1987); these references are incorporated by reference.

Antibodies produced by TRFK-5 were found to block the biological activities of both mouse IL-5 and human IL-5.

### Example II. The effects of Anti-IL-5 Antibody on Parasite-Induced Eosinophilia in Mice

In order to test the ability of an IL-5 antagonist to reduce the levels of eosinophils in various tissues, 20 Balb/c mice were treated as follows. On day zero all mice were subcutaneously injected with 800 third-stage Nippostrongylus brasiliensis larvae; e.g. see Ogilvie et al., Experimental Parasitology, vol. 29, pgs. 138-177 (1971). Five of the mice were then injected i.p. (also on day zero) with 2.0 mg of purified monoclonal antibody TRFK-5 in a phosphate buffered solution. Five of the mice as a control were injected i.p. with 2.0 mg of a purified monoclonal antibody that blocks mouse IL-4. Five of the mice as a control were injected i.p. with 2.0 mg of a purified mouse IgG₁. And five of the mice were left untreated as additional controls. On days 3, 7, 11, and 14 after injection, peripheral blood samples were taken from all mice and scored for eosinophils. The results are illustrated in Figure 1. The peripheral blood of mice treated with the IL-5-blocking antibody shows very clear reductions in eosinophils.

After day 14 the mice were sacrificed, and their lungs were removed, sectioned, fixed, and stained for eosinophils. A comparison of the numbers of eosinophils in randomly-selected microscope fields indicated that in anti-IL-5 treated mice the accumulation of eosinophils in the lung tissues was reduced by about twenty-fold.

The descriptions of the foregoing embodiments of the invention have been presented for purpose of illustration and description. They are not intended to be exhaustive, and obviously many modifications and variations are possible in light of the above teaching. The embodiments were chosen and described in order to best explain the principles of the invention and its practical application, to thereby enable others skilled in the art to best utilize the invention in various embodiments and with various modifications as are suited to the particular use contemplated. It is intended that the scope of the invention be defined by the claims appended hereto.

Applicants have deposited hybridoma TRFK-5 with the American Type Culture Collection, Rockville, MD, USA (ATCC), under accession number HB 9897. This deposit was made under conditions as provided under ATCC's Agreement for Culture Deposit for Patent Purposes.

The Deposit has been modified to satisfy the requirements of the Budapest Treaty on the Deposit of Microorganisms.

### HAT Hybrid Selection Procedures (cf. "Methods in Enzymology", Vol. LVIII, 1979, p.351-352)

HAT selection as described herein involves the use of selective medium allowing the growth of hybrid cells but killing the two parental cell types. This selection is based on the use of HAT medium, which was devised by Szybalski *et al* to kill cells that lack hypoxanthine-guanine phosphoribosyl transferase (HGPRT, EC 2.4.2.8.). The medium used by Littlefield contains hypoxanthine (0.1 mM), thymidine (16 µM), and aminopterin (0.4 µM). Aminopterin inhibits dihydrofolate reductase. Blocking the reductase inhibits *de novo* synthesis of purines and pyrimidines. The medium also must contain glycine (3 µM) because, in the presence of aminopterin, the conversion of serine to glycine is blocked (the medium is sometimes designated as GHAT medium).

Wit*h de novo* synthesis of the nucleotides blocked, cells depend on the transferase and thymidine kinase to utilize the hypoxanthine and thymine in the medium. Only cells with both of these enzymes will grow in HAT medium. Littlefield used two cell lines: one lacked thymidine kinase and was selected by growth in the presence of the toxic thymidine analog, 5'-bromodeoxyuridine; the other lacked the transferase and thus was resistant to the toxic analogs, 8-azaguanine or 6-thioguanine. Both of these parental cell types are killed in HAT medium whereas hybrids, i.e., cells containing both enzymes by complementation, will grow in HAT.

## Claims

1. The use of an antagonist to human interleukin-5 in the manufacture of a pharmaceutical composition for preventing or reducing eosinophilia in a patient.

2. The use of claim 1 wherein said antagonist to human interleukin-5 is selected from a monoclonal antibody capable of blocking the biological activity of human interleukin-5, a fragment of a monoclonal antibody capable of blocking the biological activity of human interleukin-5, and a binding composition comprising the heavy-chain variable region and light-chain variable region of a monoclonal antibody capable of blocking the biological activity of human interleukin-5.

3. The use of claim 2 wherein said fragment of said monoclonal antibody is an Fab fragment.

4. The use of claim 2 wherein said monoclonal antibody is produced by a human-human hybridoma.

5. The use of claim 4 wherein said fragment of said monoclonal antibody is an Fab fragment.

6. The use of claim 2 wherein said pharmaceutical composition is adapted for intravenous delivery of an amount of said antagonist in the range of about 1-20 mg/kg body weight of said patient per day.

## Patentansprüche

1. Verwendung eines Antagonisten für humanes Interleukin-5 bei der Herstellung einer pharmazeutischen Zusammensetzung zur Verhütung oder Verminderung der Eosinophilie bei einem Patienten.

2. Verwendung nach Anspruch, worin der Antagonist für humanes Interleukin-5 ausgewählt ist aus einem monoklonalen Antikörper, der zur Blockierung der biologischen Aktivität des humanen Interleukin-5 befähigt ist, einem Fragment eines monoklonalen Antikörpers, der die biologische Aktivität des humanen Interleukin-5 blockieren kann und einer bindenden Zusammensetzung, die einen variablen Bereich der schweren Kette und einen variablen Bereich der leichten Kette eines monoklonalen Antikörpers umfaßt, der die biologische Aktivität von humanem Interleukin-5 blockieren kann.

3. Verwendung nach Anspruch 2, worin das Fragment des genannten monoklonalen Antikörpers ein Fab-Fragment ist.

4. Verwendung nach Anspruch 2, worin der monoklonale Antikörper aus human-human Hybridom gebildet ist.

5. Verwendung nach Anspruch 4, worin das Fragment des genannten monoklonalen Antikörpers ein Fab-Fragment ist.

6. Verwendung nach Anspruch 2, worin die pharmazeutische Zusammensetzung für die intravenöse Zuführung einer Menge des Antagonisten im Bereich von ungefähr 1-20 mg/kg Körpergewicht des Patienten pro Tag angepaßt ist.

## Revendications

1. Utilisation d'un antagoniste à l'interleukine-5 humaine dans la fabrication d'une composition pharmaceutique pour prévenir ou réduire l'éosinophilie chez un patient.

2. Utilisation de la revendication 1, où ledit antagoniste à l'interleukine-5 humaine est choisi parmi un anticorps monoclonal capable de bloquer l'activité biologique de l'interleukine-5 humaine, un fragment d'un anticorps monoclonal capable de bloquer l'activité biologique de l'interleukine-5 humaine et une composition de liaison comprenant la région variable de chaîne lourde et la région variable de chaîne légère dans l'anticorps monoclonal capable de bloquer l'activité biologique de l'interleukine-5 humaine.

3. Utilisation de la revendication 2, où ledit fragment dudit anticorps monoclonal est un fragment Fab.

4. Utilisation de la revendication 2, où ledit anticorps monoclonal est produit par un hybridome humain-humain.

5. Utilisation de la revendication 4, où ledit fragment dudit anticorps monoclonal est un fragment Fab.

6. Utilisation de la revendication 2, où ladite composition pharmaceutique est adaptée à une fourniture intraveineuse d'une quantité dudit antagoniste,comprise entre environ 1-20 mg/kg de poids corporel dudit patient par jour.
